# EUROPEAN PATENT APPLICATION

(11) **EP 2 311 993 A2**
(43) Date of publication of application: **20.04.2011**
(21) Application number: 10180783.2
(22) Date of filing: 25.07.2003
(51) Int. Cl.: C12Q 1/68, C12Q 1/70

(54) **Method of detecting and quantifying hepatitis C virus**

(30) Priority: 26.07.2002 US 207693; 26.07.2002 US 398805 P
(62) Divisional of application: 09177750.8
(71) Applicant: Abbott Laboratories, Abbott Park, Illinois 60064-6008 (US)
(72) Inventor: Williams, Gregg T., Abbott Park, IL 60064-6008 (US)
(74) Representative: Modiano, Micaela Nadia

(57) **Abstract**

Methods, reagents, and kits for detecting hepatitis C virus (HCV) in biological sample.

SEQUENCE LISTING

<110> Abbott Laboratories
      Williams, Gregg T.

<120> METHOD OF DETECTING AND QUANTIFYING
  HEPATITIS C VIRUS

<130> 6874.WO.01

<140> Not Yet Assigned
<141> 2003-07-25

<160> 46

<170> FastSEQ for Windows Version 4.0

<210> 1
<211> 15
<212> DNA
<213> Artificial Sequence

<220>
<223> Forward Primer

<400> 1
tgggcgtgcc cccgc                                                        15

<210> 2
<211> 14
<212> DNA
<213> Artificial Sequence

<220>
<223> Forward Primer

<400> 2
tgggcgtgcc cccg                                                         14

<210> 3
<211> 15
<212> DNA
<213> Artificial Sequence

<220>
<223> Forward Primer

<221> misc_feature
<222> (14)...(14)
<223> N = a or g or c or t/u, unknown, or other at
      position 14

<400> 3
tgggcgtgcc cccnc                                                        15

<210> 4

<211> 15
<212> DNA
<213> Artificial Sequence

<220>
<223> Forward Primer

<221> misc_feature
<222> (11)...(11)
<223> N = a or g or c or t/u, unknown, or other at
      position 11

<400> 4
tgggcgtgcc nccgc                                                        15

<210> 5
<211> 15
<212> DNA
<213> Artificial Sequence

<220>
<223> Forward Primer

<221> misc_feature
<222> (9)...(9)
<223> N = a or g or c or t/u, unknown, or other at
      position 9

<400> 5
tgggcgtgnc cccgc                                                        15

<210> 6
<211> 15
<212> DNA
<213> Artificial Sequence

<220>
<223> Forward Primer

<221> misc_feature
<222> (7)...(7)
<223> N = a or g or c or t/u, unknown, or other at
      position 7

<400> 6
tgggcgngcc cccgc                                                        15

<210> 7
<211> 15
<212> DNA
<213> Artificial Sequence

<220>
<223> Forward Primer

<221> misc_feature
<222> (6)...(6)

<223> N = a or g or c or t/u, unknown, or other at
      position 6

<400> 7
tgggcntgcc cccgc                                                        15

<210> 8
<211> 15
<212> DNA
<213> Artificial Sequence

<220>
<223> Forward Primer

<221> misc_feature
<222> (4)...(4)
<223> N = a or g or c or t/u, unknown, or other at
      position 4

<400> 8
tggncgtgcc cccgc                                                        15

<210> 9
<211> 19
<212> DNA
<213> Artificial Sequence

<220>
<223> Forward Primer

<400> 9
tgggcgtgcc cccgcaaga                                                    19

<210> 10
<211> 18
<212> DNA
<213> Artificial Sequence

<220>
<223> Forward Primer

<400> 10
tgggcgtgcc cccgcaag                                                     18

<210> 11
<211> 17
<212> DNA
<213> Artificial Sequence

<220>
<223> Forward Primer

<400> 11
tgggcgtgcc cccgcaa                                                      17

<210> 12
<211> 16

<212> DNA
<213> Artificial Sequence

<220>
<223> Forward Primer

<400> 12
tgggcgtgcc cccgca                                                        16

<210> 13
<211> 15
<212> DNA
<213> Artificial Sequence

<220>
<223> Forward Primer

<400> 13
tgggcgtgcc cccgc                                                         15

<210> 14
<211> 19
<212> DNA
<213> Artificial Sequence

<220>
<223> Forward Primer

<221> misc_feature
<222> (15)...(15)
<223> N = a or g or c or t/u, unknown, or other at
      position 15

<400> 14
tgggcgtgcc cccgnaaga                                                     19

<210> 15
<211> 19
<212> DNA
<213> Artificial Sequence

<220>
<223> Forward Primer

<221> misc_feature
<222> (14)...(14)
<223> N = a or g or c or t/u, unknown, or other at
      position 14

<400> 15
tgggcgtgcc cccncaaga                                                     19

<210> 16
<211> 19
<212> DNA
<213> Artificial Sequence

<220>
<223> Forward Primer

<221> misc_feature
<222> (11)...(11)
<223> N = a or g or c or t/u, unknown, or other at
      position 11

<400> 16
tgggcgtgcc nccgcaaga                                                    19

<210> 17
<211> 19
<212> DNA
<213> Artificial Sequence

<220>
<223> Forward Primer

<221> misc_feature
<222> (9)...(9)
<223> N = a or g or c or t/u, unknown, or other at
      position 9

<400> 17
tgggcgtgnc cccgcaaga                                                    19

<210> 18
<211> 19
<212> DNA
<213> Artificial Sequence

<220>
<223> Forward Primer

<221> misc_feature
<222> (9)...(9)
<223> N = a or g or c or t/u, unknown, or other at
      position 9

<400> 18
tgggcggcnc cccgcaaga                                                    19

<210> 19
<211> 19
<212> DNA
<213> Artificial Sequence

<220>
<223> Forward Primer

<221> misc_feature
<222> (6)...(6)
<223> N = a or g or c or t/u, unknown, or other at
      position 6

<400> 19

tgggcntgcc cccgcaaga                                                    19

<210> 20
<211> 19
<212> DNA
<213> Artificial Sequence

<220>
<223> Forward Primer

<221> misc_feature
<222> (4)...(4)
<223> N = a or g or c or t/u, unknown, or other at
      position 4

<400> 20
tggncgtgcc cccgcaaga                                                    19

<210> 21
<211> 19
<212> DNA
<213> Artificial Sequence

<220>
<223> Forward Primer

<400> 21
tgggcgtgcc cccgcaaga                                                    19

<210> 22
<211> 22
<212> DNA
<213> Artificial Sequence

<220>
<223> Forward Primer

<400> 22
atttgggcgt gcccccgcaa ga                                                22

<210> 23
<211> 21
<212> DNA
<213> Artificial Sequence

<220>
<223> Forward Primer

<400> 23
atttgggcgt gcccccgcaa g                                                 21

<210> 24
<211> 20
<212> DNA
<213> Artificial Sequence

<220>

<223> Forward Primer

<400> 24
atttgggcgt gcccccgcaa                                                    20

<210> 25
<211> 19
<212> DNA
<213> Artificial Sequence

<220>
<223> Forward Primer

<400> 25
atttgggcgt gcccccgca                                                     19

<210> 26
<211> 18
<212> DNA
<213> Artificial Sequence

<220>
<223> Forward Primer

<400> 26
atttgggcgt gcccccgc                                                      18

<210> 27
<211> 17
<212> DNA
<213> Artificial Sequence

<220>
<223> Forward Primer

<400> 27
atttgggcgt gcccccg                                                       17

<210> 28
<211> 16
<212> DNA
<213> Artificial Sequence

<220>
<223> Forward Primer

<400> 28
atttgggcgt gccccc                                                        16

<210> 29
<211> 15
<212> DNA
<213> Artificial Sequence

<220>
<223> Forward Primer

<400> 29
atttgggcgt gcccc                                                        15

<210> 30
<211> 22
<212> DNA
<213> Artificial Sequence

<220>
<223> Forward Primer

<221> misc_feature
<222> (18)...(18)
<223> N = a or g or c or t/u, unknown, or other at
      position 18

<400> 30
atttgggcgt gcccccgnaa ga                                                22

<210> 31
<211> 22
<212> DNA
<213> Artificial Sequence

<220>
<223> Forward Primer

<221> misc_feature
<222> (17)...(17)
<223> N = a or g or c or t/u, unknown, or other at
      position 17

<400> 31
atttgggcgt gcccccncaa ga                                                22

<210> 32
<211> 22
<212> DNA
<213> Artificial Sequence

<220>
<223> Forward Primer

<221> misc_feature
<222> (14)...(14)
<223> N = a or g or c or t/u, unknown, or other at
      position 14

<400> 32
atttgggcgt gccnccgcaa ga                                                22

<210> 33
<211> 22
<212> DNA
<213> Artificial Sequence

<220>

<223> Forward Primer

<221> misc_feature
<222> (12)...(12)
<223> N = a or g or c or t/u, unknown, or other at
      position 12

<400> 33
atttgggcgt gnccccgcaa ga                                                22

<210> 34
<211> 22
<212> DNA
<213> Artificial Sequence

<220>
<223> Forward Primer

<221> misc_feature
<222> (10)...(10)
<223> N = a or g or c or t/u, unknown, or other at
      position 10

<400> 34
   atttgggcgn gcccccgcaa ga                                                 22

<210> 35
<211> 22
<212> DNA
<213> Artificial Sequence

<220>
<223> Forward Primer

<221> misc_feature
<222> (9)...(9)
<223> N = a or g or c or t/u, unknown, or other at
      position 9

<400> 35
   atttgggcnt gcccccgcaa ga                                                 22

<210> 36
<211> 22
<212> DNA
<213> Artificial Sequence

<220>
<223> Forward Primer

<221> misc_feature
<222> (7)...(7)
<223> N = a or g or c or t/u, unknown, or other at
      position 7

<400> 36
   atttggncgt gcccccgcaa ga                                                 22

<210> 37
<211> 21
<212> DNA
<213> Artificial Sequence

<220>
<223> Forward Primer

<400> 37
tttgggcgtg cccccgcaag a                                                21

<210> 38
<211> 20
<212> DNA
<213> Artificial Sequence

<220>
<223> Forward Primer

<400> 38
ttgggcgtgc ccccgcaaga                                                  20

<210> 39
<211> 19
<212> DNA
<213> Artificial Sequence

<220>
<223> Forward Primer

<400> 39
tgggcgtgcc cccgcaaga                                                   19

<210> 40
<211> 18
<212> DNA
<213> Artificial Sequence

<220>
<223> Forward Primer

<400> 40
gggcgtgccc ccgcaaga                                                    18

<210> 41
<211> 17
<212> DNA
<213> Artificial Sequence

<220>
<223> Forward Primer

<400> 41
ggcgtgcccc cgcaaga                                                     17

<210> 42

<211> 16
<212> DNA
<213> Artificial Sequence

<220>
<223> Forward Primer

<400> 42
gcgtgccccc gcaaga                                                    16

<210> 43
<211> 15
<212> DNA
<213> Artificial Sequence

<220>
<223> Forward Primer

<400> 43
cgtgcccccg caaga                                                     15

<210> 44
<211> 22
<212> DNA
<213> Artificial Sequence

<220>
<223> Reverse Primer

<400> 44
cgagacctcc cggggcactc gc                                             22

<210> 45
<211> 24
<212> DNA
<213> Artificial Sequence

<220>
<223> Probe

<400> 45
atgtgcacgg tctacgagac ctcc                                           24

<210> 46
<211> 18
<212> DNA
<213> Artificial Sequence

<220>
<223> Forward Primer

<400> 46
gccttgtggt actgcctg                                                  18

## Description

### FIELD OF THE INVENTION

The invention is directed to methods and reagents for detecting and quantifying hepatitis C virus (HCV) in biological samples and to kits for carrying out the methods.

### BIBLIOGRAPHY

Complete bibliographic citations for the documents referenced below can be found in the Bibliography section, immediately preceding the claims. All of the documents referenced below are incorporated herein by reference.

### BACKGROUND

Hepatitis C virus (HCV) is a single-stranded RNA virus, containing a genome of approximately 9,400 nucleotides. See Clark (1997). HCV has been identified as the major etiological agent for post-transfusion non-A, non-B hepatitis worldwide. Rosen & Gretch (1999). About 85% of HCV-infected individuals develop chronic hepatitis, with approximately 20% of the chronically infected individuals developing cirrhosis. See Rosen & Gretch (1999). In patients with cirrhosis, the incidence of hepatocellular carcinoma is approximately 1% to 4% per year. See Consensus Statement (1999).

Serological tests for the detection of anti-HCV antibodies have been developed. See Clark (1997). However, the presence of anti-HCV is not a direct indicator af HCV viremia, because resolved infections cannot be differentiated from active infections. Serum alanine aminotransferase (ALT) activity has been used as a surrogate indicator of active HCV infection, but ALT activity also does not provide a direct measure of HCV viremia. See, for example, U.S. Pat. No. 5,279,944.

Nucleic acid tests for detecting and quantifying HCV RNA provide a direct measure of HCV viremia, and have become increasingly important for improving blood safety, and for monitoring patients under therapy. Qualitative testing for HCV has been instrumental in reducing the number of post-transfusion cases of HCV infection. Typical of these qualitative tests is the UltraQual-brand HCV-RT-PCR Assay produced by the National Genetics Institute (Los Angeles, California). This test has been approved by the U.S. Food & Drug Administration for qualitative testing of pooled human blood plasma for the presence of HCV. See also U.S. Pat. No. 5,527,669, issued June 18, 1996.

In like fashion, quantitative tests for HCV RNA have been instrumental in understanding the effectiveness of anti-viral response to treatments such as interferon mono therapy and interferon/ribavirin combination therapy. See McHutahison et al. (1998) and Davis et al. (1998). HCV viral load testing has implications before, during, and after anti-viral therapy administration. Low levels of viremia at a baseline measurement taken prior to treatment correlated significantly with a higher response to interferon therapy, thus leading to the use of HCV viral load to predict individual patient response to such therapy. See Izopet et al. (1998); Kakumu et al. (1997); and Toyoda et al. (1996). Monitoring therapy with HCV viral load testing can enable physicians to differentiate patients who respond to treatment from those who do not, to determine the duration of treatment, and to tailor therapy for individual patients. See Izopet et al. (1998); Kakumu et al. (1997); Yamakawa et al. (1998); and Tong et al. (1997). After termination of therapy, HCV RNA levels have been used to predict relapse and to identify long term responders. Pawlotsky et al. (1996) and Chemello et al. (1996).

### SUMMARY OF INVENTION

The present invention provides improved methods, reagents, and kits for quantifying hepatitis C virus (HCV) in biological samples. The method uses any suitable method of nucleic acid amplification, and preferably uses the reverse transcriptase-polymerase chain reaction (RT-PCR), in conjunction with differential labeling and capture of the resultant amplicons, to measure quantitatively the amount of HCV RNA in a biological sample. The method uses an internal control (HCV RNA transcript that is co-amplified along with any (HCV RNA found in the sample. The internal control and any HCV RNA present in the sample are co-amplified in a competitive fashion. Thus, by comparing the amount of internal control that is amplified to the amount of HCV RNA that is amplified, the amount of HCV RNA present in a test sample can be calculated accurately and precisely. Also, because the method relies on an internal control for calibration, a calibration curve can be pre-fabricated and shipped along with the assay kit, thus limiting the number of calibration experiments the user must perform.

The preferred method generally proceeds as follows: RNA is first extracted from a biological specimen via any means now known in the art or developed in the future. In the preferred embodiment, RNA is extracted using a commercially-available kit, the "QI amp-brand Viral Nucleic Acid Extraction Kit" (Qiagen GmbH, Hilden, Germany). See "QI amp7Viral RNA Mini Kit Handbook", January 1999. The virion is lysed with a chaotrope, such as guanidiniuum isothiocyanate (GuSCN), preferably in the presence of proteinase K, thus simultaneously releasing the viral RNA and protecting it from degradation by RNAses that may be present in the sample.

At the same time the chaotrope is added to the sample, a known amount of an internal control HCV RNA transcript is also added to the sample. The internal control is identical in sequence to the HCV RNA, with the exception of a modified probe binding region that allows differential detection of the internal control. Because the internal control is identical to HCV RNA (with the exception of the unique probe binding region), the internal control has the same primer binding sites as wild-type HCV RNA.

The RNA *(i.e.,* both any HCV RNA present in the sample and the internal control) is preferably then precipitated by a suitable means now known or developed in the future (such as by adding ethanol). The RNA is then adhered to a membrane, such as a silica membrane. The adhered RNA is then washed to remove non-RNA components. Thus, for example, the RNA adhered to the membrane can be washed with a high concentration salt wash, followed by a low concentration salt wash, to remove non-RNA contaminants from the membrane. The RNA is then eluted from the membrane. It is noteworthy that in the present method, the internal control nucleic acid is co-extracted with the HCV nucleic acid present in the sample. In short, the internal control is carried through the entire method, and thus undergoes the exact same manipulations as the HCV RNA. This adds to the precision of the results generated by the method.

The isolated RNA (HCV RNA and internal control) is then co-amplified via a conventional RT-PCR protocol, preferably using the novel primers described herein. In addition to the conventional amplification reagents used in RT-PCR, to the reaction is also added two distinct labeled oligonucleotide *probes* (not primers), a *first probe* that is specific for HCV amplicons and a second *probe* that is specific for internal control amplicons. The second probe binds to the internal control amplicons via the unique probe binding region on the internal control. Thus, the RNA is first converted into a RNA-DNA heteroduplex (i.e. a cDNA strand is generated) via the action of a reverse transcriptase (preferably rTth). The cDNA is then amplified by conventional PCR to yield double-stranded DNA amplicons. The reverse transcription reaction leading to the cDNA may be catalyzed using a transcriptase that is distinct from the polymerase used in the subsequent amplification reaction. However, in the preferred embodiment, the recombinant, thermostable DNA polymerase rTth (isolated from *Thermus thermophilus)* is used. This enzyme is capable of catalyzing both the reverse transcription reaction and the subsequent amplification reaction.

The concentrations of the internal control and the primers are optimized so that the two target sequences (the HCV RNA present in the sample and the internal control) compete for primers. This competition thus forms the basis upon which the amount of HCV RNA in the test sample is determined. As the concentration of HCV RNA in the sample increases, more of the primers are used to generate HCV RNA amplicons. Simultaneously, there is a corresponding drop in the number of amplicons generated from the internal control. Thus, the ratio of patient-derived amplicon to the internal control amplicon allows the determination of the initial ratio of patient-derived HCV to internal control. Thus, the present invention provides, among other things, a method of quantifying the amount of HCV nucleic acid in a sample, such as that derived from a patient.

As noted above, in addition to the conventional amplification reagents used in RT-PCR, to the amplification reaction is also added two oligonucleotide probes: a first probe that is specific for HCV amplicons and a second probe that is specific for internal control amplicons. These probes are differentially labeled to allow them to be detected and distinguished from one another. The final heating and cooling step allows these probes to hybridize to their respective amplicons. The probes are then detected (by any means now known to the art or developed in the future) and the amount of HCV RNA amplicons and internal control amplicons are calculated based on the signals generated by the respective labels on the respective probes. As described in full in the Detailed Description, in the preferred embodiment, the probe-aanpliaon duplexes are detected using in a microparticle enzyme immunoassay (MEIA) format using an "LCx" brand automated analyzer from Abbott Laboratories.

In the preferred embodiment of the invention, one or both of the primers used to amplify both the HCV RNA and the internal control are modified to include a capture moiety, preferably a carbazole-derived hapten. See U.S. Pat. No. 5,464,746, which describes some suitable carbazole and dibenzofuran haptens for use in the present invention. In the preferred embodiment, only the reverse primer is modified to include a capture moiety.

The first and second oligonucleotide probes (i.e., the probes that are designed to hybridize to the HCV RNA amplicons and the internal control amplicons, respectively) are labeled with distinct labels that allow them to be distinguished from one another. In the preferred embodiment, one of the probes is labeled with adamantane, while the other probe is labeled with dansyl. (These are just exemplary labels. For a more detailed list of suitable labels that can be used in the invention, see the "Abbreviations and Definitions" section).

After RT-PCR and the final heating and cooling step to allow the first and second probes to hybridize to their respective amplicons, an aliquot of the amplification products is contacted with a microparticle coated with a capture reagent (such as an antibody) that recognizes the capture moiety present in the amplicons. For example, when the capture moiety on the reverse primer is a carbazole, the microparticle is coated with an anti-carbazole capture reagent (preferably an anti-carbazole antibody) that specifically recognizes and binds the carbazole moiety on the primer. In this fashion, the microparticles recognize and bind the capture moiety found in the amplification products and the free reverse primer. This serves to immobilize the amplicons (as well as unreacted primers) onto the microparticle, while leaving the labels (present only on the amplicons hybridized to that contain the first or second probe) free to react.

In the preferred embodiment, using the "LCx" brand analyzer, the microparticles are then adhered (preferably irreversibly, although irreversible binding is not required) to a glass fiber matrix. The bound microparticles are then incubated with reagents suitable to detect the different labels on the first and second probes. This is preferably done by way of a sandwich-type immunoassay. Thus, for example, where the HCV RNA probe is labeled with adamantane and the internal control probe is labeled with dansyl, the microparticle complexes are incubated with a reaction mixture containing (for example) an anti-adamantane antibody/alkaline phosphatase conjugate and an anti-dansyl antibody/β-galactosidase conjugate. The alkaline phosphatase conjugate will bind specifically to the adamantane-labeled probes (on the HCV RNA amplicons); the β-galactosidase conjugate will bind specifically to the dansyl-labeled probes (on the internal control). Adding the alkaline phosphatase substrate MUP to the reaction will result in the cleavage of the MUP and the generation of a fluorescent signal that is measured and stored. After measuring the signal, the glass fiber matrix is washed and the β-galactosidase substrate AUG is added. The β-galactosidase conjugate will cleave the AUG, thus generating a second fluorescent signal, distinct from the first. This second signal is also measured and stored. The amount of each signal is proportional to the respective amount of amplicons competitively generated from the HCV RNA and the internal control during the RT-PCR protocol. The amount of HCV RNA present in the sample can then be determined by comparing the signals generated by the test sample to a standard calibration curve. The calibration curve is generated by analyzing a series of calibration samples using the present method and generating a curve that depicts HCV concentration as a function of the signal output generated by the two labels. Each calibration sample within the series contains a fixed and known quantity of internal control. Any suitable quantity of internal control can be used. For example, as few as 1000, 500, or optionally 50 copies per calibration sample can be used in many embodiments. Similarly, as many 3,000,10,000, or optionally 30,000 copies of internal control can be used per calibration sample. The samples then vary in the amount of HCV RNA derived from the patient or sample contained in each test. Sufficient samples are tested to cover the entire dynamic detection range of the method. Thus, the number of copies of viral RNA can calculated, for example, by taking the logarithm of the ratio: {rate counts *(i.e.,* counts/second/second ("c/s/s")) for HCV RNA amplicons divided by rate counts (c/s/s) for the internal control amplicons}, and comparing the experimental value to the corresponding value on the calibration curve. In this fashion, the signal output from the test sample is converted into a value for the concentration of HCV present in the test sample.

Overall, the most preferred method according to the present invention comprises adding to a test sample suspected of containing HCV genomic nucleic acid: (i) a known amount of an internal control comprising a transcript of HCV nucleic acid; (ii) a second labeled probe specific for amplicons of the internal control oligonucleotide; and (iii) a first labeled probe specific for amplicons of HCV genomic nucleic acid. The internal control transcript and any HCV genomic nucleic acid present in the test sample are then competitively co-amplified using a forward primer and a reverse primer, thereby creating amplicons of the internal control transcript and amplicons of any HCV genomic nucleic acid present in the test sample. The test sample is then incubated under conditions that enable the first labeled probe to hybridize to the amplicons of the internal control oligonucleotide and the labeled second probe to hybridize to the amplicons of the HCV genomic nucleic acid, thereby forming hybrids. The hybrids formed between the first labeled probe and the amplicons of the internal control transcript, and the hybrids formed between the second labeled probe and the amplicons of the HCV genomic nucleic acid, are then detected. Comparing these results to a calibration curve enables the amount of HCV genomic nucleic acid present in the test sample to be quantified.

The present invention has many advantages over current means to measure HCV loads. For example, the currently available HCV assays on the market suffer from limited dynamic range and sensitivity. In contrast, the present invention includes a method to detect and quantify HCV RNA that is genotype independent and capable of precisely measuring HCV RNA levels over a dynamic range spanning five to six orders of magnitude or more. The present method is capable of accurately measuring HCV levels down to less than 100 copies per ml.

The above-noted benefits are due in large measure to the unique RT-PCR primers described herein. These primers display a highly significant increase in specificity and affinity for HCV RNA as compared to prior art primers. These primers disclosed herein quantify all of HCV genotypes 1 through 6 more consistently and without genotype bias.

The present method can also tolerate high concentrations of the competitive internal control because of the strong affinity and specificity of the forward RT-PCR primer. Thus, depending on sample volume, even when the method utilizes approximately 10,000 copies of the internal control per reaction, it will still reliably detect samples containing about 200 copies/ml of HCV RNA. Because each reaction includes a relatively high concentration of target sequences (that is, even samples devoid of HCV RNA will include, in the preferred embodiment, roughly 10,000 copies of the internal control), amplification and detection is more consistent and thus the ultimate results are far more precise than in other HCV detection formats.

Another benefit of the present method is that the reverse transcription, amplification, and oligonucleotide hybridization reactions take place in a single reaction vessel. Detection can be carried out automatically without having to open the reaction vessel manually. Because manipulation of target and product sequences is been reduced to a single-step addition, the possibility of contamination of the test sample is minimized.

Also, as discussed below, the present method can be calibrated in advance, due to the specificity of the primer and the inclusion of an internal control sequence. Thus, the signals generated from any individual test can be evaluated against the pre-fabricated calibration curve, and a HCV load value obtained.

### DETAILED DESCRIPTION OF THE INVENTION

Abbreviations and Definitions:
"Amplification reagents" designates collectively the various buffers, enzymes, primers, deoxynucleoside triphosphates, and oligonucleotides used to perform the selected PCR or RT-PCR amplification procedure.
"Amplifying" or '"Amplification" means any suitable method of amplifying a nucleic acid that employs a specific oligonucleotide probe or primer. Suitable techniques known in the art include NASBA, Transcription Mediated Amplification (TMA), Oligonucleotide Ligation Assay (OLA), Ligase Chain Reaction (LCR), and others. However, these terms preferably refer to any essentially quantitative and logarithmic increase in a target sequence as a result of a PCR designed to amplify the specific target sequence.
"Amplicon" means an amplification product.
"Annea" refers to complementary hybridization between an oligonucleotide and a target sequence and embraces minor mismatches that can be accommodated by reducing the stringency of the hybridization to achieve the desired priming for the reverse transcriptase or DNA polymerase or for detecting a hybridization signal.
"AUG" designates the substrate 7-β-galactopyranosyloxy-coumarin-4-acetic acid-(2-hydroxyethylamide). This substrate is cleaved by a β-galactosidase conjugate to yield a fluorescent signal.
"Capture reagent/capture moiety" designates any combination of compounds or moieties that specifically recognize and bind to one another. Exemplary types of capture reagent/capture moiety combinations that fall within the scope of the invention include haptens and antibodies specifically reactive with the hapten (such as carbazole and an anti-carbazole antibody). Also explicitly included are the well-known capture reactions between, for example, biotin and avidin and between glutathione and glutathione-S-transferase, etc. As used herein, the term "hapten" refers to a partial antigen or non-protein binding member which is capable of binding to an antibody, but which is not capable of eliciting antibody formation unless coupled to a carrier protein. Examples of haptens include biotin, avidin, adamantane and carbazole.
"Label" designates a molecule or moiety having a property or characteristic which is capable of detection. A label can be directly detectable, as with, for example, radioisotopes, fluorophores, chemiluminophores, enzymes, colloidal particles, fluorescent microparticles and the like; or a label may be indirectly detectable, as with, for example, specific binding members. The foregoing examples are explicitly included within the definition of "label". It will be understood that directly detectable labels may require additional components such as, for example, substrates, triggering reagents, light, and the like to enable detection of the label. When indirectly detectable labels are used, they are typically used in combination with a "conjugate". A conjugate is typically a specific binding member which has been attached or coupled to a directly detectable label. Coupling chemistries for synthesizing a conjugate are well known in the art and can include, for example, any chemical means and/or physical means that do not destroy the specific binding property of the specific binding member or the detectable property of the label. As used herein, "specific binding member" means a member of a binding pair, *i.e*., two different molecules where one of the molecules through, for example, chemical or physical means specifically binds to the other molecule. In addition to antigen- and antibody-specif binding pairs, other specific binding pairs include, but are not intended to be limited to, avidin and biotin; haptens and antibodies specific for the haptens; complementary nucleotide sequences; enzyme cofactors or substrates and enzymes; and the like.
   Many methods of adding haptens to oligonucleotide probes are known in the literature. A review of such conjugate literature is found in Goodchild (1990). Labels, haptens, and the like, can also be added to oligonucleotides as described in U.S. Pat. Nos. 5,464,746; 5,424,414; and 4,948,882.
"MEIA" refers to microparticle enzyme immunoassay. See, for example, Fiore et al. (1988), U.S. Patent Nos.: 5,358,691 (describes automated machinery for performing MEIA protocols) and 5,507,410 (describes a cartridge feeder for automated MEIA machinery). In the MEIA format, inert microparticles (usually latex beads) are covalently coupled with a capture antibody specific for a given analyte (in this case, the probe-labeled amplicons from the RT-PCR reaction). After being contacted with a sample suspected of containing the given analyte, the microparticles are then contacted with, for example, an alkaline phosphatase-antibody conjugate or a β-galactosidase-antibody conjugate. Unadsorbed materials are removed at each step by capillary action and buffer washes. Following the removal of unbound conjugate, enzyme substrates (such as MUP and/or AUG) are added sequentially and the rate of fluorescence increase is measured after each addition of substrate. In the present invention, the MEIA format is preferred. It is preferred that the MEIA be performed using automated machinery, such as Abbott Laboratories' "LCx"-brand analyzer (Abbott Laboratories, Abbott Park, Illinois). Use of automated machinery limits the likelihood of contamination of the test sample due to manual handling. The solid phase microparticle can be magnetic or non-magnetic. See, for example, published EPO applications Nos. EP 0 425 633 and EP 0 424 634.
"MUP" designates the substrate 4-methylumbelliferyl phosphate. This substrate is cleaved by an alkaline phosphatase conjugate to yield a fluorescent signal.
"Nucleic acid" refers to a deoxyribonucleotide or ribonucleotide polymer in either single- or double-stranded form, and unless otherwise limited, encompasses known analogs of natural nucleotides that can function in a manner identical to or similarly to naturally occurring nucleotides.
"Oligonucleotide" refers to a molecule comprised of two or more deoxyribonucleotides or ribonucleotides, such as primers, probes, nucleic acid fragments to be detected, and nucleic acid controls. The exact size of an oligonucleotide depends on many factors and the ultimate function or use of the oligonucleotide. Oligonuleotides can be prepared by any suitable method now known in the art or developed in the future, including, for example, using conventional and well-known nucleotide phosphoramidite chemistry and the instruments available from Applied Biosystems, Inc, (Foster City, California); DuPont, (Wilmington, Delaware); or Milligen, (Bedford, Massachusetts).
"PCR" designates the polymerase chain reaction.
"Primer" refers to an oligonucleotide, whether natural or synthetic, capable of acting as a point of initiation of DNA synthesis under conditions in which synthesis of a primer extension product complementary to a nucleic acid strand is induced, *i.e.,* in the presence of four different nucleoside triphosphates and an agent for polymerization *(i.*e., DNA polymerase or reverse transcriptase) in an appropriate buffer and at a suitable temperature. A primer is preferably a single-stranded oligodeoxyribonucleotide. The appropriate length of a primer depends on the intended use of the primer but typically ranges from 15 to 30 nucleotides and can be as short as 8 nucleotides and as long as 50 or 100 nucleotides. Short primer molecules generally require cooler temperatures to form sufficiently stable hybrid complexes with the template. A primer need not reflect the exact sequence of the template but must be sufficiently complementary to hybridize with the template.
"Probe" refers to an oligonucleotide, whether natural or synthetic, capable of hybridizing under sequence-specific, stringent conditions, to a designated target sequence. A probe need not reflect the exact sequence of the template but must be sufficiently complementary to hybridize with the template. The appropriate length of a probe depends on the intended use of the probe, and can be as short as 8 nucleotides and as long as 50 or 100 nucleotides, but typically ranges from 15 to 30 nucleotides.
"RT-PCR" designates the reverse-transcriptase-polyanerase chain reaction.
"Reverse transcriptase" refers to an enzyme that catalyzes the polymerization of deoxyribonucleoside triphosphates to form primer extension products that are complementary to a ribonucleic acid template. The enzyme initiates synthesis at the 3'-end of the primer that is annealed to the RNA template and proceeds toward the 5'-end of the RNA template until synthesis terminates. Examples of suitable polymerizing agents that convert the RNA target sequence into a complementary DNA (cDNA) sequence are avian myeloblastosis virus reverse transcriptase, *Thermococcus litoralis* (Tli) DNA polymerase, and *Thermus thermophilus* DNA polymerase (preferred), a thermostable DNA polymerase with reverse transcriptase activity. Recombinant Thermus *thermophilus* DNA polymerase (rTth) is available commercially from Applied Biosystems (catalog no. N808-0098, Foster City, California).
"Stringent conditions" designates sequence-dependent hybridization and/or amplification conditions and will be different in different circumstances. Generally, stringent conditions are selected to be about 5EC lower than the thermal melting point (Tm) for the specific sequence at a defined ionic strength and pH. The Tm is the temperature (under defined ionic strength and pH) at which 50% of the target sequences hybridizes to a perfectly matched probe. Typically, stringent conditions for PCR amplification are those wherein the buffer contains 50 mM KCI, 10 mM TrisCl, and 1.5 mM MgC1₂. Such a buffer has a pH of about 8.3 at room temperature, but the pH drops to about 7.2 at 72EC. See, e.g., Sambrook et al. (1989), Chapter 14.
"Target sequence" or "target region" are synonymous terms and designate a nucleic acid sequence (single- or double-stranded) that is detected and/or amplified, or will otherwise anneal under stringent conditions to one of the primers or probes herein provided. Target sequences can be polymorplic.
"ATest sample" designates anything suspected of containing a target sequence. The test sample can be derived from any biological source without limitation. A test sample can be used (i) directly as obtained from the source; or (ii) following a pre-treatment to modify the character of the test sample. Thus, the test sample can be pro-treated prior to use by, for example, disrupting cells and/or virions, preparing liquids from solid test samples, diluting viscous fluids, filtering liquids, distilling liquids, concentrating liquids, inactivating interfering components, adding reagents, purifying nucleic acids, and the like.
"Thermostable polymerase" refers to an enzyme that is relatively stable to heat and catalyzes the polymerization of nucleoside triphosphates to form primer extension products that are complementary to one of the nucleic acid strands of a target sequence. The enzyme initiates synthesis at the 3'-end of the primer that is annealed to the template and proceeds toward the 5'-end of the template until synthesis terminates. A purified thermostable polymerase enzyme is described more fully in U.S. Pat. Nos. 4,889,818 and 5,079,352. The term encompasses polymerases that have reverse transcriptase activity. Numerous thermostable polymerases are available from a host of commercial suppliers, such as Applied Biosystems and Promega Corporation, Madison, Wisconsin.
"Transcript" refers to a product of RNA polymerase, typically a DNA dependant RNA polymerase.

### Description:

As a general proposition, HCV genomic RNA can be detected in biological samples such as (without limitation) sera or plasma by creating cDNA from the genomic RNA, amplifying the cDNA with the polymerase chain reaction, and simultaneously or subsequently probing for the presence of the HCV cDNA amplicons with sequence-specific oligonucleotides.

Additionally, by utilizing an internal control, and subjecting the internal control to the exact same laboratory manipulations as the sample-derived HCV RNA present in the sample *(e.g.,* RNA purification, reverse transcription, amplification, immobilization on a support, etc.), the present method yields highly reliable results. In short, both the internal control and any HCV RNA present in the sample are co-analyzed in the same reaction vessel. Thus, compensation can be readily made for small deviations in sample preparation efficiency.

The basic steps of the preferred embodiment of the method are provided in the Summary of the Invention. What follows is a step-by-step discussion of each step of the method and various preferred and alternative reagents that can be used in each step.

### Specimen Preparation:

The purpose of specimen preparation is to render the specimen noninfectious, to remove any potential inhibitors of amplification from the specimen, and to concentrate the target RNA molecules and make them accessible for amplification. Any means that accomplishes these goals can be used in the present invention. For example, methods for preparing nucleic acids for RT-PCR reactions are described in Sambrook et al. (1989). Such methods for preparing RNA are well known in the art and will not be described in any further detail.

In the preferred approach, the above-noted goals are achieved by processing the specimen by protein digestion, and then separating the nucleic acids from other components present in the test sample using a commercially-available column specifically designed for this purpose. The preferred commercial product is the "Qlamp" -brand Viral Nucleic Acid Extraction Kit, from Qiagen, GmbH. For a full discussion of the kit and how it is used, see the "QI amp7 Viral RNA Mini Kit Handbook", dated January 1999.

Briefly, the "QI amp" -brand kit utilizes the binding properties of a silica-based "QI amp" -brand spin column to isolate viral RNA and DNA from sample lysates in the presence of denaturing reagents. Test samples are lysed using specially formulated reagents to disrupt viral particles, free nucleic acids, and protect RNA and DNA from digestion by nucleases. The products from test sample lysis (lysates) are transferred to "QI amp"-brand spin columns for nucleic acid binding. Once bound to the "QI amp" column matrix, the nucleic acids are purified using wash buffers to remove contaminants which inhibit the PCR. A vacuum system, such as the "LCx"-brand vacuum system from Abbott Laboratories (Abbott Park, Illinois) is used for the processing of sample lysates and wash reagents through the columns. Alternatively, the various wash reagents can be moved through the columns using centrifugation. The "LCx"-brand vacuum system is preferred because it can process up to 48 samples simultaneously. Purified nucleic acids are eluted with water by centrifuging the "QI amp" column. Viral nucleic acids are recovered in the eluate, free from proteins, nucleases, and other contaminants. After being eluted from the "QI amp" column, the purified nucleic acids from the test sample (both the HCV RNA and the internal control) are ready for amplification without further purification or preparation.

When the amplification reaction is performed by PCR, the PCR reaction is preferably impeded until after the first denaturation step. Suitable methods for achieving this include, but are not limited to, keeping the reaction components cold until heating the reaction in the first denaturation step, using an enzyme that is only activated by exposure to high temperatures, and adding the enzyme to the reaction in the presence of a (non-heat stabile) antibody that binds to the enzyme and inhibits polymerization.

### Primers:

As noted above, the primers to be used in the present method are preferably from 15 to 30 nucleotides long and must specifically prime the reverse transcription and amplification of only the HCV RNA and the internal control present in the test sample.

The present inventors have found that forward primers containing any of the sub-sequences (or complements thereof) recited in Table 1 are extraordinarily specific for HCV genomic RNA:

**TABLE 1: Preferred Forward Primers**

| | | | | | | | | |
|---|---|---|---|---|---|---|---|---|
| SEQ. ID. NO: 1 | | TGG | GCG | TGC | CCC | CGC | | |
| SEQ. ID. NO: 2 | | TGG | GCG | TGC | CCC | CG | | |
| SEQ. ID. NO: 3 | | TGG | GCG | TGC | CCC | CNC | | |
| SEQ. ID. NO: 4 | | TGG | GCG | TGC | CNC | CGC | | |
| SEQ. ID. NO: 5 | | TGG | GCG | TGN | CCC | CGC | | |
| SEQ. ID. NO: 6 | | TGG | GCG | NGC | CCC | CGC | | |
| SEQ. ID. NO: 7 | | TGG | GCN | TGC | CCC | CGC | | |
| SEQ. ID. NO: 8 | | TGG | NCG | TGC | CCC | CGC | | |
| SEQ. ID. NO: 9 | | TGG | GCG | TGC | CCC | CGC | AAG | A |
| SEQ. ID. NO: 10 | | TGG | GCG | TGC | CCC | CGC | AAG | |
| SEQ. ID. NO: 11 | | TGG | GCG | TGC | CCC | CGC | AA | |
| SEQ. ID. NO: 12 | | TGG | GCG | TGC | CCC | CGC | A | |
| SEQ. ID. NO: 13 | | TGG | GCG | TGC | CCC | CGC | | |
| SEQ. ID. NO: 14 | | TGG | GCG | TGC | CCC | CGN | AAG | A |
| SEQ. ID. NO: 15 | | TGG | GCG | TGC | CCC | CNC | AAG | A |
| SEQ. ID. NO: 16 | | TGG | GCG | TGC | CNC | CGC | AAG | A |
| SEQ. ID. NO: 17 | | TGG | GCG | TGN | CCC | CGC | AAG | A |
| SEQ. ID. NO: 18 | | TGG | GCG | GCN | CCC | CGC | AAG | A |
| SEQ. ID. NO: 19 | | TGG | GCN | TGC | CCC | CGC | AAG | A |
| SEQ. ID. NO: 20 | | TGG | NCG | TGC | CCC | CGC | AAG | A |
| SEQ. ID. NO: 21 | | TGG | GCG | TGC | CCC | CGC | AAG | A |
| SEQ. ID. NO: 22 | ATT | TGG | GCG | TGC | CCC | CGC | AAG | A |
| SEQ. ID. NO: 23 | ATT | TGG | GCG | TGC | CCC | CGC | AAG | |
| SEQ. ID. NO: 24 | ATT | TGG | GCG | TGC | CCC | CGC | AA | |
| SEQ. ID. NO: 25 | ATT | TGG | GCG | TGC | CCC | CGC | A | |
| SEQ. ID. NO: 26 | ATT | TGG | GCG | TGC | CCC | CGC | | |
| SEQ. ID. NO: 27 | ATT | TGG | GCG | TGC | CCC | CG | | |
| SEQ. ID. NO: 28 | ATT | TGG | GCG | TGC | CCC | C | | |
| SEQ. ID. NO: 29 | ATT | TGG | GCG | TGC | CCC | | | |
| SEQ. ID. NO: 30 | ATT | TGG | GCG | TGC | CCC | CGN | AAG | A |
| SEQ. ID. NO: 31 | ATT | TGG | GCG | TGC | CCC | CNC | AAG | A |
| SEQ. ID. NO: 32 | ATT | TGG | GCG | TGC | CNC | CGC | AAG | A |
| SEQ. ID. NO: 33 | ATT | TGG | GCG | TGN | CCC | CGC | AAG | A |
| SEQ. ID. NO: 34 | ATT | TGG | GCG | NGC | CCC | CGC | AAG | A |
| SEQ. ID. NO: 35 | ATT | TGG | GCN | TGC | CCC | CGC | AAG | A |
| SEQ. ID. NO: 36 | ATT | TGG | NCG | TGC | CCC | CGC | AAG | A |
| SEQ. ID. NO: 37 | TT | TGG | GCG | TGC | CCC | CGC | AAG | A |
| SEQ. ID. NO: 38 | T | TGG | GCG | TGC | CCC | CGC | AAG | A |
| SEQ. ID. NO: 39 | | TGG | GCG | TGC | CCC | CGC | AAG | A |
| SEQ. ID. NO: 40 | | GG | GCG | TGC | CCC | CGC | AAG | A |
| SEQ. ID. NO: 41 | | G | GCG | TGC | CCC | CGC | AAG | A |
| SEQ. ID. NO: 42 | | | GCG | TGC | CCC | CGC | AAG | A |
| SEQ. ID. NO: 43 | | | CG | TGC | CCC | CGC | AAG | A |

where N can be A, C, T, or G.

From among these sub-sequences, the preferred forward primers are those containing a sub-sequence (or complement thereof) as shown in SEQ. ID. NO: 1, SEQ. ID. NO: 22, SEQ. ID. NO: 30, and SEQ. ID. NO: 35.

The nature of the reverse primer is less critical to the operation of the subject invention than is the forward primer. Thus, as a general proposition, the invention will function and yield acceptable results with any reverse primer that will operate in conjunction with the forward primer to amplify HCV RNA and the internal control specifically. It is preferred, however, that the combination of forward and reverse primers chosen yield an amplification product that is at least 20 and preferably less than 250 nucleotides long.

The preferred reverse primers for use in PCR based embodiments of the present invention are:
SEQ. ID. NO: 44: CGAGACCTCC CGGGGCACTC GC, and
SEQ. ID. NO: 45: ATGTGCACGG TCTACGAGAC CTCC.

The forward primer is preferably unlabeled with the reverse primer modified to include a capture reagent, such as (without limitation) carbazole, on its 5 terminus or end.

### Internal Control Nucleic Acid:

The internal control is a transcript of HCV genomic nucleic acid, preferably taken from the 5' untranslated region (*utr*) of the HCV genome. The internal control is modified to include a unique probe binding region that is complementary in sequence to the probe that is specific for amplicons of the internal control. In all other respects, however, the internal control is identical in sequence to a portion of the HCV genome. Thus, the internal control has the same primer binding sites as the corresponding location within the HCV genomic nucleic acid itself.

Adverse variability in the efficiency of the reverse transcription and amplification reactions can be compensated for in the present method because the internal control and the HCV RNA are co-reverse transcribed and co-amplified. Thus, the internal control experiences the exact same conditions as the HCV RNA during reverse transcription and amplification.

The present method utilizes two distinct oligonucleotide probes: a second probe that hybridizes specifically to amplicons of the internal control and a first probe that hybridizes specifically to amplicons of the HCV genomic RNA. Both probes are modified to include a detectable label *(i.e.,* a hapten), such as adamantane or dansyl. The probes must be labeled with different haptens so that they can be distinguished from one another in the detection phase of the assay.

The preferred probe for HCV RNA amplicons is:
SEQ. ID. NO: 46: GCC TTG TGG TAC TGC CTG.

### Labels and Capture Reagents (Haptens):

As noted above, the two probes used in the invention are differentially labeled to allow them to be detected and distinguished from one another. Detection of the label can be by either direct or indirect means. The exact nature of the label is not critical to the overall functionality of the invention, so long as the label does not interfere with the hybridization of the probes to their respective amplicons and are not deleterious to any of the other components of the assay. The two differentially-detectable labels that are most preferred for use in the present invention are adamantane-derived labels (designated collectively herein as simply "adamantine") and dansyl. Preferred adamantane labels are described in U.S. Pat. No. 5,424,414.

Affixing such labels to oligonucleotides is known in the art. For suitable labeling methodologies, see, e.g., U.S. Pat. Nos. 5,464,746; 5,424,414; and 4,948,882.

Some preferred capture reagents are described in U.S. Pat. No. 5,464,746.

### Amplification:

RT-PCR amplification, preferably using the labeled primers listed above, proceeds in standard fashion.

The target sequence for the amplification within the internal control and the genomic HCV RNA present in the sample is within the 5¹ *utr* region of HCV genome. This region is specific for HCV and is highly conserved across all known HCV genotypes. The preferred primers are designed so that they will hybridize to the target region within the 5' *utr* with the fewest possible mismatches among the various HCV genotypes. This minimizes genotype bias, thereby enabling the present method to detect and quantify accurately all known HCV genotypes. As discussed herein, the reverse primer is preferably labeled with a moiety, while the HCV-specific probe is labeled with a first label, and the internal control-specific probe is labeled with a second label.

The reverse transcription reaction may be performed separately from the amplification reaction. However, it is preferred that the two reactions be performed as a single protocol using a polymerase that includes reverse transcriptase activity, such as thermostable rTth.

Therefore, in the preferred embodiment, the thermostable polymerase has a dual enzymatic function: it serves as both a reverse transcriptase and a DNA-dependant DNA polymerase. During an extended incubation period, the reverse transcriptase activity generates a cDNA extension product from the reverse primer which has hybridized to the RNA target (the HCV RNA and the internal control). This cDNA product then acts directly as a template during PCR amplification. The second primer is complementary to a region of the cDNA product and acts as a primer for PCR extension from the cDNA product.

During thermalcycling, the temperature of the reaction is raised above the melting point of the hybridized product, causing the strands to dissociate. Lowering the temperature allows the excess primers to anneal to the dissociated strands and generate new products. Exponential amplification of the products is achieved through repeated cycling between high and low temperatures. Forty-five thermal cycles are sufficient to generate a billion-fold or greater amplification in target sequences. Amplification of both targets (HCV if present, and the internal control) takes place simultaneously in the same reaction.

An additional cycle with a high-temperature denaturation and low temperature incubation allows the HCV-specific probe and internal control-specific detection probe to anneal to their respective amplicons.

A suitable mixture of amplification reagents includes the following ingredients in the concentrations specified: 50 mM bicine, 115 mM K⁺, 150 µM dNTPs, 125 nM capture moiety-modified reverse primer, 50 nM forward primer, internal control (∼10,000 copies per RT-PCR), 50 nM labeled HCV nucleic acid-specific probe, 50 nM labeled internal control nucleic acid-specific probe, 2.5 units rTth polymerase, 0.01 mg/ml acetylated BSA, 0.02% sodium azide, 8% glycerol, 0.5 mM MnCl₂, 0.225% sodium azide, and 0.005% xylenol orange dye, pH 8.25, and optionally 0.3125% Tween-20.

A typical set of thermalcycling conditions to effect both the reverse transcription and amplification reaction is as follows:

| | |
|---|---|
| cDNA synthesis: | 94EC, 1 min; 62EC, 30 min; 94EC, 2 min; I cycle. |
| Amplification: | 94EC, 1 min; 62EC, 1 min; 45 cycles. |
| Probe hybridization: | 97EC, 5 min; 15EC, 5 min; 1 cycle. |

Where the thermalcycling equipment used is more fully programmable, the following conditions are preferred:

| | |
|---|---|
| cDNA synthesis: | 94EC, 1 min; 62EC, 30 min; 94EC, 2 min; 1 cycle. |
| Amplification: | 94EC, 15 sec; 58EC, 20 sec; with 1 sec extension per |
| | cycle; 5 cycles; followed by 94EC, 15 sec; 62EC, 25 see; |
| | with 1 sec extension per cycle; 40 cycles. |
| Probe hybridization: | 97EC, 5 min; 15EC, min; 1 cycle. |

Amplicons can soak in the amplification reagent mixture at 15EC for up to 96 hours prior to further analysis.

The primers and internal control are optimized such that the two targets (HCV genomic RNA and the internal control) compete for primers. Thus, the anapliftcation protocol is designated as being competitive. When the concentration af HCV genomic RNA increases as compared to the amount of internal control, there is a corresponding drop in the number of amplicons generated from the internal control. The ratio of the amount of amplicon generated from the internal control versus the amount af amplicon generated from HCV genomic RNA is then used to quantify the amount of HCV genomic RNA that was present in the test sample.

### Detection:

After the amplification reaction is complete and the hybridization of the HCV RNA and internal control probes is complete, the sample is ready for detection. In the last step of the amplification protocol, the HCV probe and the internal control probe hybridize to their respective amplification products during the final cycle when the reaction is cooled below the melting temperature of the detection probes. Because the detection probes are complementary to internal sequences of the target strands, they add specificity to the assay by eliminating spurious amplification products (such as primer dimers) from detection. The two-probe format design allows dual detection of both the HCV and internal control amplicons.

Thus, in the preferred embodiment, the HCV probe and the internal control probe are differentially labeled and one or both of the *primers* (preferably the reverse primer only) is modified to include a capture moiety. In the detection step, the amplicons and unreacted primers are immobilized on an inert support coated with a capture reagent to facilitate further analysis of the amplicons. Thus, for example, when the reverse primer is modified to include a carbazole capture moiety, the inert support, preferably a microparticle is coated with anti-earbazole antibody (rabbit). Note that both the amplicons and the unreacted primers that include the capture moiety are immobilized on the microparticle. However, because the unreacted primers will not be detected in the subsequent detection step (the primers are not labeled), this does not affect the outcome of the analysis.

An aliquot of the amplification product is transferred to a reaction well containing the coated microparticles. The coated microparticles recognize and bind to the capture moiety found on the amplification product and the unreacted free reverse primers. In the preferred embodiment, using the LCx"-brand analyzer, the reaction mixture is then automatically transferred to a glass fiber matrix to which the microparticle complexes bind irreversibly. After washing, the bound microparticle complexes are incubated with reagents that allow the amount of HCV amplicons and internal control amplicons to be detected and quantified. This is done by detecting the differential labels present on the HCV probes and the internal control probes.

In the preferred embodiment, the HCV probe is labeled with adamantane and the internal control probe is labeled with dansyl. To detect these labels, antibodies that are specific to the labels are conjugated to enzymes that can be directly detected. The conjugates are then contacted with the microparticles, whereby the conjugates will bind to their respective labels. Adding enzyme substrate will then yield distinct signals for the HCV amplicons and distinct signals for the internal control amplicons. These two values are then compared to yield a ratio by which the amount of HCV nucleic acid in the original test sample is determine.

For example, to detect the HCV probe (which, in this illustration, is labeled with adamantane), an anti-adamantane antibody is conjugated to (for example) an alkaline phosphatase enzyme. The anti-adamantane/alkaline phosphatase conjugate will bind only to the HCV-specific probe. The microparticle are incubated with the anti-adamantane/alkaline phosphatase conjugate under conditions and for a time sufficient for the antibody portion of the conjugate to bind to the adamantane probe. Then, the alkaline phosphatase substrate MUP is added to the incubation reaction. This causes a reaction wherein the MUP substrate is cleaved by the alkaline phosphatase portion of the conjugate and a fluorescent product is produced. The fluorescent product is detected by well known optical means for detecting fluorescence, such as a photometer. The magnitude of the fluorescent signal is proportional to the amount of bound HCV amplicon.

In the same fashion, to detect the internal control probe (which, in this illustration, is labeled with dansyl), an anti-dansyl antibody is conjugated to (for example) a β-galactosidase enzyme. The anti-dansyl/β-galactosidase conjugate will bind only to the internal control-specific probe. The microparticles are incubated with the anti-dansyl/ β-galactosidase conjugate under conditions and for a time sufficient for the antibody portion of the conjugate to bind to the dansyl probe. Then, the β-galactosidase substrate AUG is added to the incubation reaction. This causes a reaction wherein the AUG substrate is cleaved by the β-galactosidase portion of the conjugate and a fluorescent product is produced. The fluorescent product is detected by well know optical means for detecting fluorescence, such as a photometer. The magnitude of this second fluorescent signal is proportional to the amount of bound internal control amplicon.

The order of probe detection is not critical. The nature of the specific labels on the probes is also not critical to the function of the invention, with three caveats:
1. The labels preferably do not interfere with the reverse transcription or amplification reactions.
2. Whatever two labels are chosen (one for the HCV probe, one for the interval control probe), they must be different from one another and differentially detectable so that the two signals can be distinguished from one another.
3. The chemistry required to detect the two different labels preferably do not interfere with one another.

With these three caveats being satisfied, the present invention can be practiced with any two differentially-detectable labels, including radioactive, fluorophoric, chromophoric, and enzymatic labels.

Conjugating the antibody so produced to an enzyme, such as alkaline phosphatase, is accomplished by means well known to the art. By way of example, alkaline phosphatase can be conjugated to an antibody according to the following scheme:
1. Dialyze 5 mg/ml antibody solution against 2 L of 0.1 M PBS (pH 6.8) at 4EC.
2. Remove antibody solution from dialysis membrane and adjust to 3 mg/ml with PBS.
3. Add 100 µ1 dialyzed antibody solution to 90 µl alkaline phosphatase in a 1.5 ml centrifuge tube.
4. Add 5 ml glutaraldehyde and mix gently. Let stand at room temperature.
5. Remove 25 µ1 samples at time 0, 5, 10,15, 30, 60, and 120 min and place in separate 1.5 ml microfuge tubes. Add 125 µl PBS to each sample, and then add 1.1 ml Tris/ovalbumin solution. Store each sample on ice until the time course is completed.
6. Dialyze the samples against PBS as described in step 1. Test each sample for alkaline phosphatase activity to determine which conjugation time yields the most active enzyme conjugate.
7. Repeat steps 1 to 4, but in step 4 allow the reaction to proceed for the optimal conjugation time, as determined in step 6.
8. Add sodium azide to 0.1% and store the conjugate protected from light at 4EC. It should remain active for up to a year. Alternatively, add an equal volume of glycerol and store the conjugate frozen at -20EC.

### Calibration Curves:

One of the distinct advantages of the present invention is that because the internal control is carried through the entire procedure, from RT-PCR, through isolation and analysis of the amplicons, me method can be calibrated *a priori* using a set of calibration standards. Thus, the numbers of calibration runs that must be performed by the end user are minimized. In short, all the user needs to do is perform the assay, calculate the logarithm of the ratio: {rate counts *(i.e.,* c/s/s) for HCV RNA amplicons) by rate counts (c/s/s) for the internal control amplicons}, and compare the obtained value to a corresponding pre-fabricated calibration curve to arrive at the concentration of HCV in the test sample.

The calibration samples used to compile a suitable calibration curve each contain a fixed and known concentration of internal control (e.g., 10,000 copies per RT-PCR). The calibration samples also contain systezrzatically-varied levels of HCV transcript that span the entire dynamic range of the method. Thus, an exemplary series of calibration samples would include the following samples (2,000 or 10,000 copies of internal control per sample):

**TABLE 2: Calibration Samples**

| Calibrator Mix No. | log copies HCV/ml | copies HCV/mL |
|---|---|---|
| 1 | --- | 0 |
| 2 | 2.30 | 200 |
| 3 | 3.30 | 2,000 |
| 4 | 4.30 | 20,000 |
| 5 | 5.30 | 200,000 |
| 6 | 7.30 | 20,000,000 |

Several different sets of calibration samples were assembled and tested using the preferred method. The HCV transcripts used to fabricate the calibration samples were matched to World Health Organization candidate reference materials obtained from the Sanquin Blood Supply Foundation, Plesmanlaan 125, 1066CX Amsterdam, The Netherlands (formerly known as the CLB).

Thus, for example, in reiterative testing (n > 100) of the present method against a battery of calibration samples wherein each sample contained 10,000 copies of the internal control and 0, log 2.50, log 3.02, log 3.59, log 4.09, log 6.15 and log 7.19 copies/ml of HCV transcript, the following standard deviations were obtained:

**TABLE 3: Standard Deviations for Calibration Samples**

| SAMPLE TESTED | STANDARD DEVIATION (n > 100) |
|---|---|
| present method at 0 copies HCV per ml | 0.428 |
| present method at log 2.50 copies HCV per ml | 0.173 |
| present method at log 3.02 copies HCV per ml | 0.133 |
| present method at log 3.59 copies HCV per ml | 0.042 |
| present method at log 4.09 copies HCV per ml | 0.050 |
| present method at log 6.15 copies HCV per ml | 0.029 |
| present method at log 7.19 copies HCV per ml | 0.027 |

Note that a three standard deviation-window surrounding the 0 calibration sample and the log 2.5 calibration sample do not overlap. Thus, negative samples are easily discerned from those "low positive" samples, *i.e.,* those samples containing approximately log 2.50 copies of HCV per ml.

In a study using serum plasma taken from 10 known HCV negative patients, each patient provided three samples (a serum sample, an EDTA plasma sample, and an ACD plasma sample). Each sample was spiked with log 3.00 copies HCV/ml and then tested according to the preferred embodiment of the present method. The standard deviation for these 30 samples was 0.116, a value that comports quite closely with the 0.133 standard deviation seen in the log 3.02 copies/ml calibrator sample from Table 3.

Negative serum samples and "low-positive" serum samples (log 3.5 HCV copies/ml) were also spiked with potentially interfering compounds to see if these compounds would disrupt the accurate measurement of HCV in the sample. Adding protein (90 mg/ml total - 55 mg/ml albumin and 35 mg/ml gamma globulin), hemoglobin (10 mg/ml), bilirubin (0.4 mg/ml), lipids (30 mg/ml and 20% liposyn II), or HCV anti-viral drugs (273 lU/ml interferon α-2b or a combination af 273 IU/ml interferon α-2b and 0.05 µg/ml ribavirin) to the samples had no significant effect on the, results generated from the spiked samples as compared to controls.

### Kits:

The present invention also encompasses kits that include all or a sub-set of the primers, probes, labels, reagents, etc., required to practice the subject method. Thus, the preferred embodiment of the kit would comprise one or more vessels containing the forward and reverse primers, the internal control, the HCV-specifia probe, and the internal control-specific probe. These components could be delivered dissolved in suitable buffers or lyophilized. The kit may optionally include instructions for practicing the HCV detection method. The kits may optionally include a pre-fabricated calibration curve.

Other embodiments of the kits would include a far more extensive collection of reagents, such as buffers, dNTPs, rTth enzyme, stop reagents, conjugate reagent mixtures, etc. These ingredients would be disposed in a plurality of containers to make the practice of the subject method more convenient for the end user. Thus, such an illustrative kit according to the present invention can include the following mixtures, each disposed in its own container:
1. 2x Amplification Reagent Mix: 100 mM bicine, 230 mM K+, 300 µM dNTPs, 250 nM reverse primer, 100 nM forward primer having a sequence consisting essentially of SEQ ID NO: 1-43,100 nM labeled HCV nucleic acid-specific probe, 100 nM labeled internal control nucleic acid-specific probe, 5 units rTth polymerase, 0.02 mg/ml acetylated BSA, 0.04% sodium azide, 16% glycerol, internal control (10,D00 copies per RT-PCR) (pH 8.25). This is diluted 100°/a to yield the lx amplification reagent mix.
2. Activation Reagent Mix: 10 mM MnC1₂, 0.45% sodium azide, O.Q 1 Q % xylenol orange dye. Adding the activation reagent mix to the Ix amplification reagent mix initiates the RT-PCR reaction. Thus, this mix is not added until the samples are ready for amplification.
3. Internal Control Nucleic Acid Reagent Mix: Alternatively, the internal control RNA transcript (10,000 copies per RT-PCR) (listed above in the Amplification Reagent Mix) can be included as a separate component. The internal control would the be provided in a solution of 10 mM HEPES, 0.2 mM EDTA,100 µg/ml acetylated BSA, 10 mM DTT, 0.5 U/µ1 RNAses inhibitor, 8725 µg/ml poly-A, 0.045% sodium azide.
4. Anti-carbazale Microparticles: 0.400 µn-diameter particles coated with rabbit anti-carbazole antibodies in a solution of 0.265% tris (hydroxymethyl) amino methane
5. Conjugate Reagent Mix: anti-dansyl/β-galactosidase conjugate and anti-adamantane/alkaline phosphatase conjugate; each ∼Iµg/ml with respect to antibody
6. MUP (1.2 mM in buffer) and AUG (3.0 mM in buffer), enzyme substrates for alkaline phosphatase and β-galactosidase, respectively.

### Further Embodiments of the Invention:

The present invention first relates to a compound comprising an isolated oligonucleotide of from 15 to 30 nucleotides, wherein the oligonucleotide contains anywhere within it a sub-sequence selected from the group consisting of SEQ. ID. NOS: 1-43 and complements thereof.

In one embodiment of such compound, the sub-sequence is selected from the group consisting of SEQ. ID. NOS: 1-8 and complements thereof.

In another embodiment of such compound, the sub-sequence is selected from the group consisting of SEQ. ID. NOS: 9-21 and complements thereof.

In still another embodiment of such compound, the sub-sequence is selected from the group consisting of SEQ. ID. NOS: 22-43 and complements thereof.

The above oligonucleotide may be labeled. The above oligonucleotide may be labeled with a hapten.

The invention also relates to a method of quantifying an amount of hepatitis C virus (HCV) genomic nucleic acid in a test sample, the method comprising:
(a) adding to a test sample suspected of containing HCV genomic nucleic acid:
   (i) a known amount of an internal control oligonucleotide comprising an HCV nucleic acid sequence; (ii) a first labeled probe specific for amplicons of the internal control oligonucleotide; and (iii) a second labeled probe specific for amplicons of HCV genomic nucleic acid; then
(b) competitively co-amplifying the internal control oligonucleotide and any HCV genomic nucleic acid present in the test sample from step (a) using a forward primer having a nucleic acid sequence selected from the group consisting of SEQ ID NO: 1-43 and complements thereof and a reverse printer, thereby creating amplicons of the internal control oligonucleotide and amplicons of any HCV genomic nucleic acid present in the test sample; thin
(c) incubating the test sample from step (b) under conditions that enable the first labeled probe to hybridize to the amplicons of the internal control oligonucleotide and the labeled second probe to hybridize to the amplicons of the HCV genomic nucleic acid, thereby forming hybrids; and then (d) detecting the hybrids formed between the first labeled probe and the amplicons of the internal control oligonucleotide and the hybrids formed between the second labeled probe and the amplicons of the HCV genomic nucleic acid, whereby the amount of HCV genomic nucleic acid present in the test sample is quantified.

In one embodiment of the method, in step (a), the first labeled probe comprises an oligonucleotide consisting of SEQ ID NO: 46 and complements thereof.

In another embodiment of the method, the forward primer has a nucleic acid sequence selected from the group consisting of SEQ ID NOS: 1-8 and complements thereof.

The present invention also relates to a kit for quantifying an amount of hepatitis C virus (HCV) genomic nucleic acid in a test sample, the kit comprising in combination:
a first vessel containing a primer pair capable of specifically amplifying HCV genomic nucleic acid, one primer being selected from the group consisting of SEQ ID NOS: 1-44 and complements thereof;
a second vessel containing an internal control HCV oligonucleotide, the internal control HCV oligonucleotides comprising a nucleic acid transcript from the 5' untranslated region of the HCV genome; and
a third vessel containing a first probe specific for amplicons of HCV genomic RNA, and a second probe specific for amplicons of the internal control HCV oligonucleotide.

### BIBLIOGRAPHY

Chernello, Cavaletto, & Casarin, et al. (1996) "Persistent hepatitis C viremia predicts relapse after sustained response to interferon-α in chronic hepatitis C";'Ann. Intern. Med. 124; 1058-60.
Clarke (1997) "Molecular virology of hepatitis C virus", J. Gen. Virol. 78:2397-2410.
Consensus Statement (1999) "ASL International Consensus Conference on Hepatitis C", patol. 30:356-61.
"Current Protocols in Molecular Biology", vol. 2, Chapter 11, supplement 53, 8 2001, John Wiley and Songs, Inc.
Fiore et al. (1988) Clin. Chem. 34:1726-1732.
McHutchison, Gordon, & Stuart, et al. (1998) "nterferon alpha-2b alone or in combination with ribavirin as initial treatment for chronic hepatitis C. International Hepatitis Interventional Therapy Group", Engl. J. Med. 339:1485-92.
Davis, Esteban Mur, & Rustgi, et al, (1998) "nterferon alpla-2b alone or in combination with ribavirin for the treatment of relapse of chronic hepatitis C. International Hepatitis Interventional Therapy Group", N. Eng. J. Med. 339:1493- 99.
Goodchild (1990) Bioconjugate Chem. 1(3):165-187.
Izopet, Payen, & Alric, et al. (1998) "Baseline level and early suppression of serum HCV RNA for predicting sustained complete response to alpha-interferon therapy", J. Med. Virol. 54:86-91.
Kakumu, Toshiyuki, & Okumura, et al. (1997) "Earlier loss of hepatitis C virus RNA in interferon therapy can predict a long term response in chronic hepatitis C", J. Gastroenterol. Hepatol. 12:468-72.
Langone & Van Vukis, eds. (1986) "Immunological techniques, Part I: Hybridoma technology and monoclonal antibodies", Meth. Enzymal. 121:1-947.
Myers TW, Gelfand DH. Reverse transcription and DNA amplification by a Thermus thermophilus DNA polymerase. Biochem 1991 ;30:7661 6.
Pawlotsky, Roudot-Thoraval, & Bastie A, et al. (1996) "Factors affecting treatment responses to interferon-α in chronic hepatitis C", J. Infect. Dis. 174:1-7.
Rosen & Gretch (1999) "Hepatitis C virus: Current understanding and prospects for future therapies", Mol. Med. Today 5:393-99*.*
Sambrook, Fritsch, Maniatis (1989) "Molecular CloningXA Laboratory Manual Second Edition", Cold Spring Harbor Laboratory Press, Cold Spring Harbor, NY 8 1989).
Tong, Blatt, & McHutchison, et al. (1997) "Prediction of response during interferon alpha-2b therapy in chronic hepatitis C patients using viral and biochemical characteristics: A comparison", Hepatol. 26:1640-45.
Toyoda, Nakano, & Kumada, et al. (1996) "Comparison of serum hepatitis C virus RNA concentration by branched DNA probe assay with competitive reverse transcription polymerase chain reaction as a predictor of response to interferon-alpha therapy in chronic hepatitis C patients", J. Med. Virol. 48:354-59.
Van Vukis and Langone, eds. (1980) "lmmunochemical techniques", Meth. Enzymol. 70:1-525.
Yamakawa, Sata, & Suzuki (1998) "Monitoring of serum levels of HCV RNA in early phase of IFN therapy; as a predictive marker of subsequent response", Hepato-Gastroenterol. 45:133-36.

## Claims

1. A primer selected from the group consisting of (1) a forward primer comprising an isolated oligonucleotide of from 15 to 30 nucleotides, wherein the oligonucleotide contains anywhere within it a sub-sequence selected from the group consisting of: SEQ ID NO: 1, SEQ ID NO:30, SEQ ID NO:35 and complements thereof or (2) a reverse primer having a sequence selected from the group consisting of: SEQ ID NO:44, SEQ ID NO:45 and complements thereof.

2. The primer of claim 1, wherein the primer is labeled.

3. The primer of claim 1, wherein the primer is labeled with a hapten.

4. A method of quantifying an amount of hepatitis C virus (HCV) genomic nucleic acid in a test sample, the method comprising:
a) adding to a test sample suspected of containing HCV genomic nucleic acid: (i) a known amount of an internal control oligonucleotide comprising an HCV nucleic acid sequence; (ii) a first labeled probe specific for amplicons of the internal control oligonucleotide; and (iii) a second labeled probe specific for amplicons of HCV genomic nucleic acid; then
b) competitively co-amplifying the internal control oligonucleotide and any HCV genomic nucleic acid present in the test sample from step (a) using a forward primer having a nucleic acid sequence selected from the group consisting of: SEQ ID NO: 1, SEQ ID NO:30, SEQ ID NO:35 and complements thereof and a reverse primer, thereby creating amplicons of the internal control oligonucleotide and amplicons of any HCV genomic nucleic acid present in the test sample; then
c) incubating the test sample from step (b) under conditions that enable the first labeled probe to hybridize to the amplicons of the internal control oligonucleotide and the labeled second probe to hybridize to the amplicons of the HCV genomic nucleic acid, thereby forming hybrids; and then
d) detecting the hybrids formed between the first labeled probe and the amplicons of the internal control oligonucleotide and the hybrids formed between the second labeled probe and the amplicons of the HCV genomic nucleic acid, whereby the amount of HCV genomic nucleic acid present in the test sample is quantified.

5. The method of claim 4, wherein the reverse primer is selected from the group consisting of: SEQ ID NO:44, SEQ ID NO:45 and complements thereof

6. A kit for quantifying an amount of hepatitis C virus (HCV) genomic nucleic acid in a test sample, the kit comprising in combination:
a primer pair capable of specifically amplifying HCV genomic nucleic acid, one primer being selected from the group consisting of SEQ ID NO: SEQ ID NO: 1, SEQ ID N0:30, SEQ ID NO:35, and complements thereof;
an internal control HCV oligonucleotide, the internal control HCV oligonucleotide comprising a nucleic acid transcript from the 5' untranslated region of the HCV genome; and
a first probe specific for amplicons of HCV genomic RNA, and a second probe specific for amplicons of the internal control HCV oligonucleotide,

7. The kit of claim 6, further comprising a primer selected from the group consisting of SEQ ID NO:44, SEQ ID N0:45 and complements thereof.
